# EUROPEAN PATENT SPECIFICATION

(11) **EP 3 614 943 B1**
(45) Date of publication and mention of the grant of the patent: **01.01.2025**
(21) Application number: 18790978.3
(22) Date of filing: 16.03.2018
(51) Int. Cl.: A61B 18/12, A61B 18/04, A61B 18/14, A61B 18/00, A61N 1/05, A61N 1/40, A61N 1/32

(54) **ENHANCED ELECTROPORATION OF CARDIAC TISSUE**
VERBESSERTE ELEKTROPORATION VON HERZGEWEBE
ÉLECTROPORATION AMÉLIORÉE DE TISSU CARDIAQUE

(30) Priority: 24.04.2017 US 201715495537
(43) Date of publication of application: 04.03.2020
(60) Divisional application: 24212473.3
(73) Proprietor: Medtronic Cryocath LP, Toronto, Ontario M5L 1B9 (CA)
(72) Inventor: STEWART, Mark T., Lino Lakes, Minnesota 55014 (US); HOWARD, Brian T., Hugo, Minnesota 55038 (US); FRAASCH, Steven J., Maple Grove, Minnesota 55311 (US)
(74) Representative: FRKelly
(86) International application number: PCT/CA2018/050324
(87) International publication number: WO 2018/195650

(56) References cited:
- EP-A1- 1 171 189
- WO-A1-2015/175570
- WO-A1-2017/024123
- WO-A2-2016/161201
- US-A- 6 023 638
- US-A1- 2016 166 310
- US-B2- 8 116 874

## Description

### TECHNICAL FIELD

The present invention relates to a device and system for treating cardiac arrhythmia. In particular, the present invention relates to a system for enhancing lesion formation without arrhythmogenic effects within relatively thick target tissues, such as the ventricles of the heart.

### BACKGROUND

Cardiac arrhythmias can often be corrected by means of ablation, or destroying, of those cardiac cells that originate, support, or otherwise contribute to the propagation of errant conduction pathways or signals in the heart that ultimately interfere with normal cardiac electrical activity and function. For example, arrhythmias in specific cases such as the relatively thin tissue of the atrium may manifest as atrial fibrillation, or, when present in or involving the thicker tissue of the ventricles, may cause other serious conditions such as ventricular tachycardia.

Of the various forms of energy that may be delivered to accomplish tissue ablation, exposure of tissue to pulsed electric fields to produce irreversible electroporation (IRE) is one of the fastest and most controlled. The use of this energy depends on the type of tissue being targeted for ablation and the pulse parameters may need to be tailored specifically to accomplish the desired result. While IRE is effective, it is limited to tissue encompassed by the isopotential surface extent of the electric field threshold for tissue death.

Other forms of ablation such as radiofrequency (RF) use more energy to raise the temperature of the target cells and surrounding tissue until the target cells die. This can be associated with undesired thermal effects outside or even within the target region. However, at even a sub-lethal level of temperature increase, the biological processes associated with these thermal ablations have other desirable characteristics. For example, with the application of heat through RF energy, the cell membranes become more energetic and dynamic with proteins becoming more mobile and ultimately the membranes may in general become easier to disrupt.

In the presence of charge delivery to an electrode, the effect in biological mediums within a cell or the extracellular fluid can be the creation of chemical species capable of inducing cellular damage or death with varying degrees of effect and longevity. The types and total effect can vary according to the manner in which the electrical energy is applied.

Additionally, although the use of pulsed field ablation (PFA) to induce IRE is generally safe and effective, in some cases the voltage required to be applied to the tissue-contacting electrodes may be unacceptably high, due to potential loss of electrical isolation on conductors within the treatment device, which may lead to failure of the device from short circuits and ohmic heating of the conductors within the device.

Documents WO 2015/175570 A1, EP 1 171 189 A1 and WO 2016/161201 A2 disclose relevant background art.

### SUMMARY

The invention is defined in the appended independent claim. Embodiments of the invention are disclosed in the dependent claims.

The methods described herein do not form part of the invention.

This disclosure provides a method of multiple waveform energy delivery to perform a series of sequential functions on the tissue targeted for ablation. This may include high-amplitude short-pulse-duration biphasic charge-neutral pulses followed by or combined with a series of lower amplitude non-charge-neutral pulses delivered between or in conjunction with the high amplitude pulses. These lower-amplitude pulses may be monophasic or biphasic with a DC offset, such that they impart current flow through the affected tissue. In any case, the pulses should be very short, less than 50 microseconds in pulse duration and preferably less than about 20 microseconds to avoid induction of arrhythmias. Within such tissues, this DC energy between anode and cathode will produce changes in acid-base balance along with active oxygen species at the anode that will enhance and increase targeted cell death. Such a system will include charge monitoring from the power generator to determine microcoulombs of charge that are imparted to the tissue. Similar but lower-amplitude non-charge-neutral deliveries may also be used to stun tissue. Extremely short, low-amplitude pulses have the potential to impart charge to a targeted tissue site without causing ablation but having the effect of stunning or briefly causing inactivation of these excitable cells.

In one embodiment, a medical system includes: a first treatment device; a second treatment device; and an energy generator in communication with the first and second treatment devices, the energy generator being programmed to: deliver charge-neutral pulses; and deliver non-charge-neutral pulses between the charge-neutral pulses.

In one aspect of the embodiment, the energy generator is further programmed to deliver the charge-neutral pulses at a first amplitude and the non-charge-neutral pulses at a second amplitude, the first amplitude being greater than the second amplitude.

In one aspect of the embodiment, the non-charge-neutral pulses are one of monophasic and biphasic.

In one aspect of the embodiment, the non-charge-neutral pulses have a direct current offset. In one aspect of the embodiment, the system is configured to deliver charge-neutral and non-charge neutral pulses to an area of target tissue, delivery of the non-charge-neutral pulses imparting a charge to the target tissue. In one aspect of the embodiment, the energy generator is further programmed to deliver non-charge-neutral pulses at a third amplitude, the third amplitude being less than each of the first and second amplitudes.

In one aspect of the embodiment, each of the first and second treatment devices includes at least one treatment electrode that is configured to be inserted into an area of target tissue.

In one aspect of the embodiment, the at least one treatment electrode is a needle-shaped electrode.

In one aspect of the embodiment, the at least one treatment electrode is a helical-shaped electrode.

In one aspect of the embodiment, the at least one treatment element is in fluid communication with a fluid source, the at least one treatment element including a plurality of apertures configured to deliver fluid from the fluid source to the area of target tissue.

In one aspect of the embodiment, the energy generator is further programmed to deliver pulsed radiofrequency energy one of concurrently with or independently from the delivery of the non-charge-neutral pulses and the charge-neutral pulses. In one aspect of the embodiment, the pulsed radiofrequency energy is one of unipolar and bipolar, the pulsed radiofrequency energy being delivered for a predetermined period of time before the delivery of the non-charge-neutral pulses and the charge-neutral pulses, the predetermined period of time being sufficient to heat the tissue to a temperature that is lower than a temperature at which tissue ablation occurs.

In one embodiment, a medical system includes: a treatment device including: an elongate body having a proximal portion and a distal portion defining a distal tip; a first electrode, the first electrode defining the distal tip; and a second electrode being configured to at least partially puncture an area of tissue, the second electrode extending distally from the first electrode; and an energy generator in communication with the treatment device, the energy generator being programmed to: deliver charge-neutral pulses through the second electrode, the second electrode being configured to be an anodic electrode during the delivery of non-charge-neutral pulses; deliver non-charge-neutral pulses between the charge-neutral pulses from the second electrode; deliver pulsed radiofrequency energy through the first electrode one of concurrently with and independently from delivery of the non-charge-neutral pulses and the charge-neutral pulses; establish a predetermined charge level; calculate a total amount of charge delivered to the target tissue, the total amount of charge being based on a number and duration of the delivered non-charge-neutral pulses; and automatically adjust delivery of the non-charge-neutral pulses to maintain the predetermined charge level.

In one embodiment, a method for delivering energy to an area of target tissue includes: positioning a first treatment device at a first location relative to the area of target tissue; positioning a second treatment device at a second location relative to the area of target tissue; delivering biphasic charge-neutral pulses between the first and second treatment devices at a first amplitude; and delivering non-charge-neutral pulses between the first and second treatment devices at a second amplitude.

In one aspect of the embodiment, the first amplitude is greater than the second amplitude.

In one aspect of the embodiment, the first amplitude is greater than the second amplitude non-charge-neutral pulses are one of monophasic and biphasic.

In one aspect of the embodiment, the first location is within a first chamber of the patient's heart in contact with endocardial tissue and the second location is within a second chamber of the patient's heart proximate the first location.

In one aspect of the embodiment, the first location is within a chamber of the patient's heart in contact with endocardial tissue and the second location is within a pericardial space around the patient's heart.

In one aspect of the embodiment, the first location is within a chamber of the patient's heart in contact with endocardial tissue and the second location is within one of a coronary arterial blood vessel and a venous blood vessel.

In one aspect of the embodiment, the method further includes, before delivering biphasic charge-neutral pulses between the first and second treatment devices at the first amplitude, delivering energy between the first and second treatment devices, the energy being sufficient to heat cardiac tissue to a temperature of between 45 °C and 60 °C.

In one embodiment, a method for electrolyzing an area of target tissue may include positioning a treatment device having a plurality of electrodes in contact with an area of target tissue, delivering biphasic energy pulses between at least two of the plurality of electrodes to at least one of stun and ablate cells within the area of target tissue, and then delivering at least one of monophasic energy pulses and continuous direct current to the cells within the area of target tissue to ablate the cells within the area of target tissue.

### BRIEF DESCRIPTION OF THE DRAWINGS

A more complete understanding of the present invention, and the attendant advantages and features thereof, will be more readily understood by reference to the following detailed description when considered in conjunction with the accompanying drawings wherein:
FIG. 1 shows a first exemplary medical system including a first treatment device and a second treatment device, each treatment device having an invasive electrode configured to be inserted into tissue;
FIG. 2 shows a second exemplary medical system including a first treatment device and a second treatment device, the second treatment device having an invasive electrode configured to be inserted into tissue;
FIG. 3 shows a third exemplary medical system including a first treatment device and a second treatment device, each treatment device having electrodes that are not configured to be inserted into tissue;
FIG. 4 shows a fourth exemplary medical system including a single treatment device, the treatment device having an invasive electrode configured to be inserted into tissue;
FIG. 5 shows a fifth exemplary medical system including a single treatment device, the treatment device having electrodes that are not configured to be inserted into tissue;
FIG. 6 shows a sixth exemplary medical system including a single treatment device, the treatment device having an invasive electrode configured to be inserted into tissue and an electrode that is not configured to be inserted into tissue;
FIG. 7 shows exemplary biphasic energy pulses and monophasic pulses delivered thereafter for inducing electrolysis;
FIG. 8 shows exemplary pulsed RF energy delivery to warm tissue, then biphasic energy pulses and monophasic pulses delivered thereafter for inducing electrolysis;
FIG. 9 shows exemplary monophasic energy pulses;
FIG. 10 shows exemplary monophasic energy pulses with concurrent delivery of direct current (DC);
FIG. 11 shows exemplary biphasic energy pulses with a DC offset for inducing electrolysis;
FIG. 12 shows further exemplary monophasic energy pulses with concurrent delivery of DC;
FIG. 13 shows exemplary asymmetric biphasic energy pulses for inducing electrolysis;
FIG. 14 shows exemplary biphasic energy pulses in which the relative anode versus cathode is reversed;
FIG. 15 shows a flow chart for a first exemplary method of delivering energy to tissue;
FIG. 16 shows a flow chart for a second exemplary method of delivering energy to tissue;
FIG. 17 shows a flow chart for a third exemplary method of delivering energy to tissue;
FIG. 18 shows a flow chart for a fourth exemplary method of delivering energy to tissue;
FIG. 19 shows a first example of device position; and
FIG. 20 shows a second example of device position.

### DETAILED DESCRIPTION

The devices, systems, and methods disclosed herein are for ablating tissue using a multiple waveform energy delivery that may include high amplitude short pulse duration biphasic charge-neutral pulses combined or sequenced with a series of lower amplitude non-charge neutral pulses delivered between or in conjunction with the high amplitude pulses. These lower amplitude pulses may be monophasic or biphasic with a DC offset, such that they impart current flow through the affected tissue. In any case, the pulses should be very short, less than 50 microseconds in pulse duration and preferably less than about 20 microseconds to avoid induction of arrhythmias. Within such tissues, this DC energy between anode and cathode will produce changes in acid-base balance along with active oxygen species at the anode that will enhance and increase targeted cell death. Such a system will include charge monitoring from the power generator to determine micro coulombs of charge that are imparted to the tissue. Similar but lower amplitude non charge neutral deliveries may also be used. Extremely short, biphasic, high-amplitude, high-voltage, charge-neutral pulses may be delivered to ablate or incapacitate an area of tissue, and may also have the effect of stunning tissue beyond the area of ablation. Non-charge-neutral, low-amplitude, monophasic energy may then be delivered to the same area of tissue to further ablate or incapacitate the tissue. Delivering non-charge-neutral energy may have arrhythmogenic effects; however, this energy may be delivered safely due to the inactivation of the tissue by the previously delivered short, biphasic, high-amplitude energy. Further, toxic compounds generated by the non-charge-neutral energy may extend the area of ablation even deeper into the tissue. The method may also include optimizing tissue for electroporation by the delivery of energy, such as relatively low voltage AC pulsed currents, continuous RF, and/or pulsed RF or microwave energy, to heat the tissue before, during, or after delivering monophasic and/or biphasic electroporation energy.

Before describing in detail exemplary embodiments that are in accordance with the disclosure, it is noted that components have been represented where appropriate by conventional symbols in drawings, showing only those specific details that are pertinent to understanding the embodiments of the disclosure so as not to obscure the disclosure with details that will be readily apparent to those of ordinary skill in the art having the benefit of the description herein.

As used herein, relational terms, such as "first," "second," "top" and "bottom," and the like, may be used solely to distinguish one entity or element from another entity or element without necessarily requiring or implying any physical or logical relationship or order between such entities or elements. The terminology used herein is for the purpose of describing particular embodiments only and is not intended to be limiting of the concepts described herein. As used herein, the singular forms "a", "an" and "the" are intended to include the plural forms as well, unless the context clearly indicates otherwise. It will be further understood that the terms "comprises," "comprising," "includes" and/or "including" when used herein, specify the presence of stated features, integers, steps, operations, elements, and/or components, but do not preclude the presence or addition of one or more other features, integers, steps, operations, elements, components, and/or groups thereof.

Unless otherwise defined, all terms (including technical and scientific terms) used herein have the same meaning as commonly understood by one of ordinary skill in the art to which this disclosure belongs. It will be further understood that terms used herein should be interpreted as having a meaning that is consistent with their meaning in the context of this specification and the relevant art and will not be interpreted in an idealized or overly formal sense unless expressly so defined herein.

In embodiments described herein, the joining term, "in communication with" and the like, may be used to indicate electrical or data communication, which may be accomplished by physical contact, induction, electromagnetic radiation, radio signaling, infrared signaling or optical signaling, for example. One having ordinary skill in the art will appreciate that multiple components may interoperate and modifications and variations are possible of achieving the electrical and data communication. In addition, the term "in fluid communication with" may be used to describe a fluid pressure or flow connection between points, such as a fluid connection on the handle of a device that delivers fluid through a passage in the catheter to an electrode or distal site on the device.

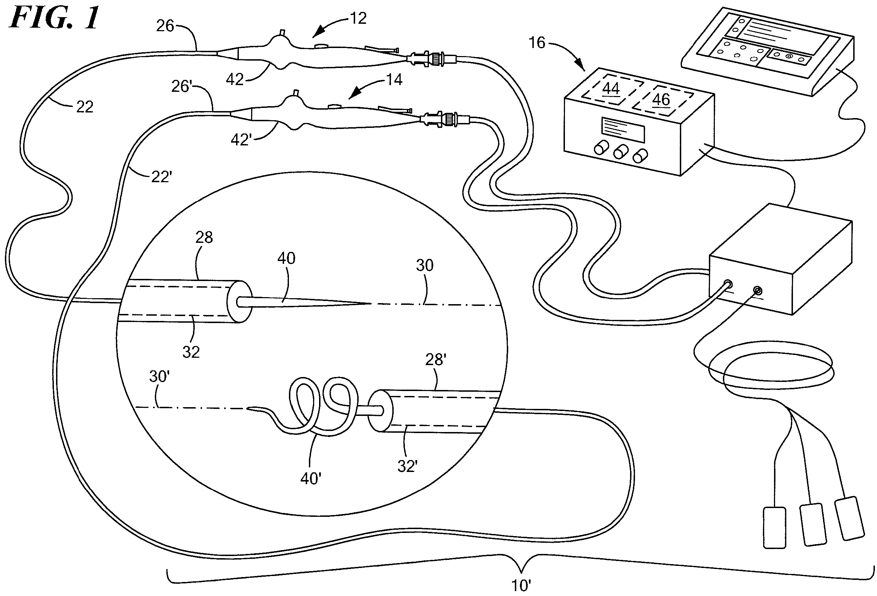

Referring now to the drawing figures in which like reference designations refer to like elements, an embodiment of a medical system is shown in FIG. 1, generally designated as "10." The device components have been represented where appropriate by conventional symbols in the drawings, showing only those specific details that are pertinent to understanding the embodiments of the present invention so as not to obscure the disclosure with details that will be readily apparent to those of ordinary skill in the art having the benefit of the description herein. Moreover, while certain embodiments or figures described herein may illustrate features not expressly indicated on other figures or embodiments, it is understood that the features and components of the system and devices disclosed herein are not necessarily exclusive of each other and may be included in a variety of different combinations or configurations.

As noted above, the effectiveness of IRE may depend on the isopotential surface extent of the electric field threshold for tissue death. When performing electroporation, there is a region nearest the delivering electrodes that may experience an electrical field sufficient to cause cell death. Beyond that is a region that has been subjected to electroporation, but at a level that is reversible and, therefore, the cells will restore their function. Beyond that reversibly electroporated region is tissue that only experiences minimal, if any, effects from the applied field. Within this secondary region, where the cells have increased permeability for treatment, a pulsing routine such as described below capable of producing toxic chemical species both within the cells and intracellular space can expand the permanently ablated region. The regions with increased permeability allow for the uptake of these damaging chemical species and can reduce the survival of the reversible region and potentially beyond. This can for example allow for an ultimately deeper and/or larger lesion for treating arrhythmias in thicker cardiac tissue or during irreversible ablation of cells in other target tissue.

When applying electric pulses or electric currents to the excitable cells of the cardiac myocardium, care must be taken to avoid generation of arrhythmias or aberrant cardiac conduction. Methods by which this may be accomplished include: 1) the delivery of extremely short pulses that have a pulse width and amplitude which is insufficient to produce cardiomyocyte depolarization; 2) initial delivery of charge-neutral (or balanced charge) biphasic pulses to stun and/or ablate tissue before delivering monophasic or continuous direct current; 3) delivery of localized charge imbalances with an overall charge balance maintained in the heart; 4) periodically alternating the electrodes serving as anode versus cathode in order to alternate the type of chemistry created at each electrode; and 5) a system to monitor and control the amount of charge delivered to the tissue with automation to adjust the level of pulse imbalance to maintain a desired or predetermined charge level.

Additional means may be employed to enhance the effectiveness of such enhanced ablations. One preferred method may include delivery of electrodes to within the distal arterial circulation to add cytotoxic electrolysis products to the blood supply being delivered to the targeted tissues to enhance the ablative effect of electric field exposure. In addition, dual electrode bipolar helix or needle electrodes may be employed to produce localized lethal chemical milieu deeper within the ventricular myocardium to enhance the ablative effects of electric field exposure.

In addition to or instead of the production of toxic chemical species, the target tissue may be heated prior to, during, and/or after the delivery of electroporation energy in order to enhance the effectiveness of the electroporation procedure. For example, tissue may be heated by delivering relatively low voltage AC pulsed currents, continuous radiofrequency, and/or pulsed radiofrequency or microwave energy before, during, and/or after the delivery of electroporation energy.

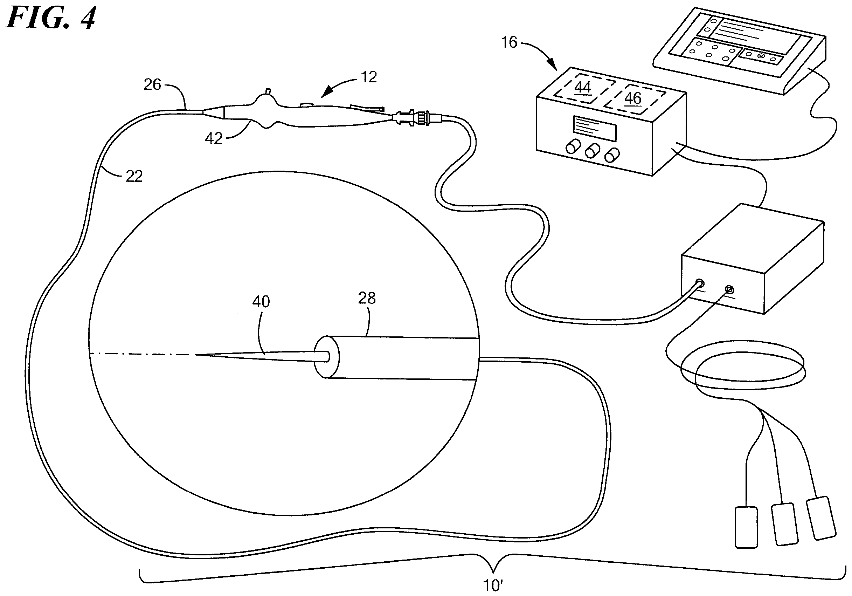

One embodiment of the system 10 may generally include a first treatment device 12 and a second treatment device 14 in communication with a control unit or energy source, such as a radiofrequency pulsed field ablation energy generator 16 (for example, as shown in FIGS. 1-3). In another embodiment, the system 10 may generally include a single treatment device 12 and a generator 16 (for example, as shown in FIGS. 4-6). The generator 16 may be configured to deliver both high amplitude short pulse duration biphasic charge-neutral pulses and lower amplitude non-charge neutral pulses delivered. In one embodiment, the generator 16 may additionally be configured to deliver pulsed radiofrequency energy sufficient to heat tissue to a target temperature.

Each of the first 12 and second 14 treatment devices (or, alternatively, the single treatment device 12) may include an elongate body 22, 22' passable through a patient's vasculature and/or proximate to a tissue region for diagnosis or treatment. For example, the treatment device(s) 12, 14 may be catheters that can access various cardiac locations, such as the atria, the ventricles, and/or the pericardial space, by such delivery means as femoral, radial, and/or sub-xiphoid access. Each elongate body 22, 22' may define a proximal portion 26, 26', a distal portion 28, 28', and a longitudinal axis 30, 30', and may further include one or more lumens disposed within the elongate body 22, 22' that provide mechanical, electrical, and/or fluid communication between the elongate body proximal portion 26, 26' and the elongate body distal portion 28, 28'. For example, the elongate body 22, 22' may include a central lumen 32, 32'.

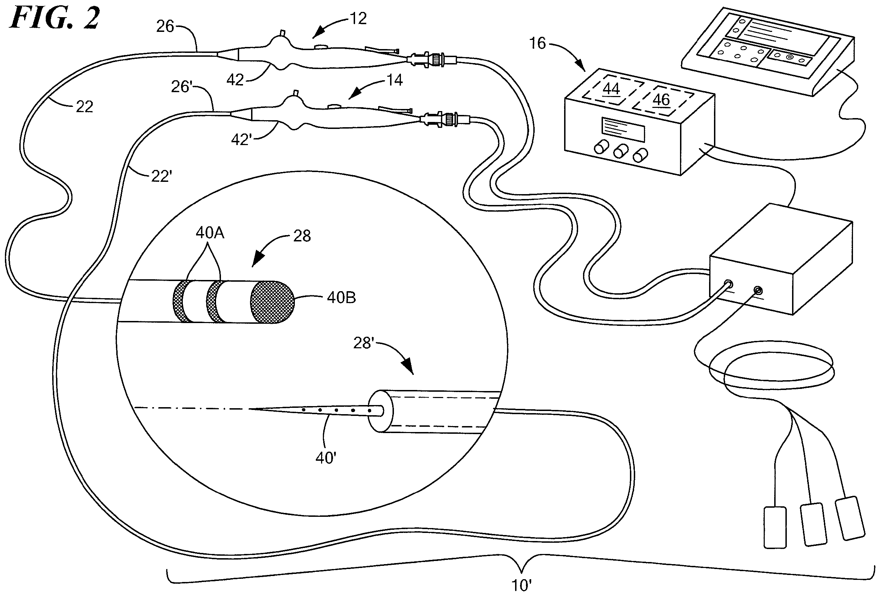

In the non-limiting system example shown in FIG. 1, each treatment device 12, 14 may include at least one electrode 40 that is configured to at least partially puncture or be inserted into myocardial tissue (which may be referred to herein as an invasive electrode). The at least one invasive electrode 40 may be configured to be retracted into and extended from the distal end of the elongate body or may be in a fixed position at the distal end of the elongate body. As a non-limiting example, the first treatment device 12 may include a needle-like or needle-shaped energy delivery electrode 40 and the second treatment device 14 may include a helical-shaped energy delivery electrode 40' that is configured to be rotated (or screwed) into the target tissue (for example, as shown in FIG. 1). Alternatively, the first treatment device 12 may include the helical energy delivery electrode 40 and the second treatment device 14 may include the needle-like energy delivery electrode 40', or both treatment devices 12, 14 may have the same type of electrode 40, 40'. Further, other suitable types of electrodes may be used for delivering the energy in the manner discussed herein. For example, each device may include one or more electrodes 40 that are configured for the delivery of treatment energy but that are not configured to be embedded or inserted within tissue, which may be referred to herein as "flat" electrodes 40A (as shown in FIGS. 2, 3, and 5). Additionally, each device may also include a distal tip electrode 40B (as shown in FIGS. 2, 3, 5, and 6). When referring to electrodes in general, rather than specifically to 40', 40A, 40A', 40B, or 40B', reference number "40" may be used herein for simplicity. The electrode(s) 40 may be composed of any suitable material, such as one or more electrically conductive metals, including gold, platinum, platinum-iridium alloy, tantalum, silver, silver-chloride, and/or tantalum with various forms of tantalum oxide surfaces. Although the distal ends 28, 28' of the devices 12, 14 are shown as having a fixed diameter (for example, focal catheters), it will be understood that the electrodes 40 may additionally or alternatively be on an expandable portion of the device(s), such as an expandable balloon.

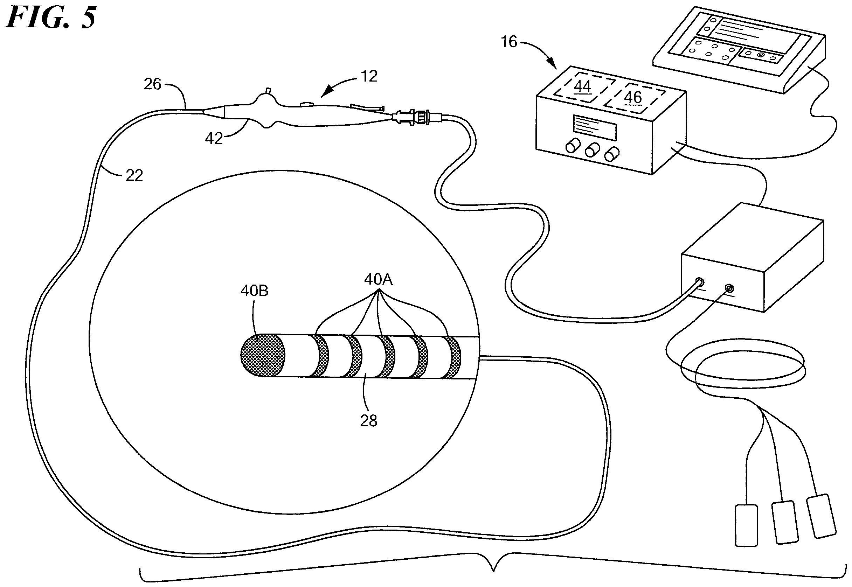

When only one device 12 is used to deliver energy (for example, the device shown in FIG. 5), the device 12 may include more than one electrode 40, and adjacent electrodes 40 may have opposite polarities. However, when the intent is to create electrochemical products of the direct electric current, some physical separation may be maintained between the electrode(s) serving as the anode(s) and the electrode(s) serving as the cathode(s), such that the chemical moieties created at the anode(s) remain relatively contained within the targeted region of tissue and unable to readily mix with the chemical milieu created at the cathodic electrode(s). This may allow for the greatest tissue cytotoxic effect from the locally created chemicals. Additionally, one or both anode and cathode electrodes may be embedded in some way within the tissue. For example, if the electrode 40 is in the form of a needle (such as that shown in FIGS. 1 and 2), the electrode 40 may be able to penetrate into the tissue, or an electrode of the device 12 may be delivered into the lumen of a distal artery where the blood perfusion flow targets the desired area to be ablated.

Further, each invasive electrode may be configured to deliver hypertonic solution, such as a hypertonic and ionic solution, and/or a solution containing a high calcium concentration, to the target treatment site to enhance the formation of lethal chemical byproducts at the site. As a non-limiting example, at least one invasive electrode 40 may be in fluid communication with a fluid source, the fluid source containing one or more hypertonic and ionic solutions, and the at least one invasive electrode 40 may have one or more orifices or apertures 41 through which the solution may pass to the tissue site (for example, as shown in FIG. 2).

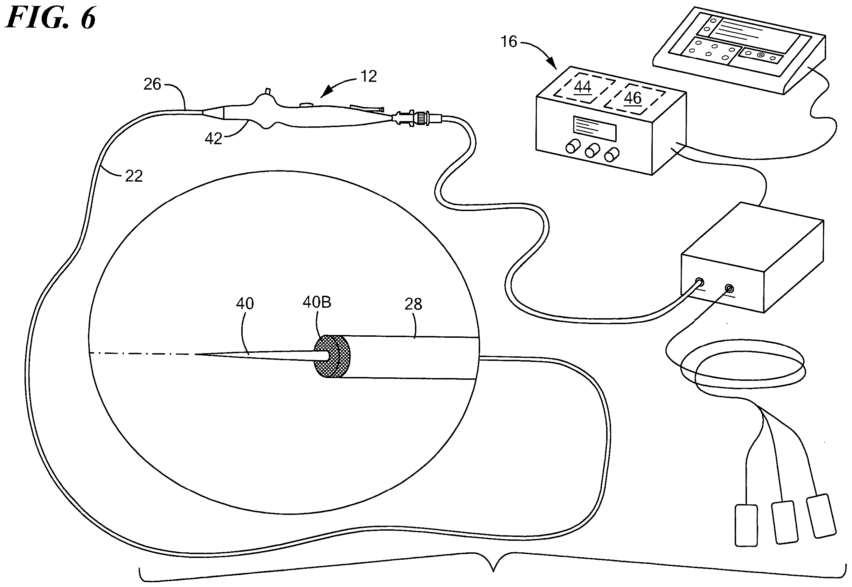

Still further, each device 12, 14 may include more than one type of electrode for delivering more than one type of energy. As a non-limiting example, the system may include a single device 12 that includes both an invasive electrode 40 and a distal tip electrode 40B (as shown in FIG. 6). As a non-limiting example, the flat electrode 40B may define the distal tip of the device and the invasive electrode 40 may extend distally from the flat electrode 40B. A device having such a configuration may be able to deliver both pulsed RF energy with the distal tip electrode 40B and pulse combinations (for example, high-voltage, short-pulse-duration, biphasic, charge-neutral pulses combined with a series of lower-amplitude, non-charge-neutral pulses delivered between the high-amplitude pulses) with the invasive electrode 40. The flat electrode 40B may also be able to deliver energy for heating the tissue, such as relatively low voltage AC pulsed currents, continuous RF, and/or pulsed RF or microwave energy. The invasive electrode 40 and the distal tip electrode 40B may be activated to deliver energy simultaneously, in series, or in an alternative fashion during a single treatment procedure.

Each device 12, 14 may include a handle 42 coupled to the elongate body proximal portion 26. Each handle 42 may include circuitry for identification and/or use in controlling of the treatment device(s) 12, 14 or another component of the system 10. Additionally, the handle 42 may also include connectors that are mateable to the control unit 16 to establish communication between the device(s) 12, 14 and one or more components or portions of the control unit 16. The handle 42 may also include one or more actuation or control features that allow a user to control, deflect, steer, or otherwise manipulate a distal portion of the device(s) 12, 14 from the proximal portion of the device. For example, the handle 42 may include one or more components such as a lever or knob for manipulating the elongate body 22 and/or additional components of the device(s) 12, 14.

The system 10 may include other components such as a navigation system, an imaging system, or other system components or add-on components for collecting and conveying information from and to the user, for delivering treatment energy to the patient, and/or for collecting data from the tissue or other parts of the patient and/or system. The generator 16 may include one or more controllers, software modules, and/or processing circuitry 44 configured to execute instructions or algorithms to provide for the automated operation and performance of the features, sequences, calculations, or procedures described herein and/or required for a given medical procedure. In one embodiment, the processing circuitry 44 may include a processor and a memory. The memory may be in electrical communication with the processor and have instructions that, when executed by the processor, configure the processor to receive, process, or otherwise use signals from the device(s) 12, 14. Further, the processing circuitry 44 may include a charge monitoring device 46 to calculate a total amount of change (for example, in microcoulombs) that is delivered to the tissue.

Although not shown, the system 10 may include one or more sensors to monitor the operating parameters throughout the system, including for example, pressure, temperature, flow rates, volume, power delivery, impedance, pH level, or the like in the control unit 16 and/or the treatment device(s) 12, 14, in addition to monitoring, recording or otherwise conveying measurements or conditions within the device(s) 12, 14 and/or the ambient environment at the distal portion of the device(s) 12, 14. The sensor(s) may be in communication with the control unit 16 for initiating or triggering one or more alerts or therapeutic delivery modifications during operation of the device(s) 12, 14. One or more valves, controllers, or the like may be in communication with the sensor(s) to provide for the controlled dispersion or circulation of fluid through the lumens/fluid paths of the device. Such valves, controllers, or the like may be located in a portion of the device(s) and/or in the control unit 16.

The generator 20 may be configured to deliver high-voltage, high-amplitude, short-pulse-duration, biphasic, charge-neutral pulses combined with a series of lower-amplitude, non-charge-neutral pulses delivered between the high-amplitude pulses. This delivery scheme may be referred to herein as a pulse combination. Such pulse combinations may include the delivery of single high-amplitude biphasic pulses of short pulse duration (of, for example, a duration of 5 µS), each followed by a low-amplitude monophasic pulse (of, for example, a duration of 100 mS) or a series of low-amplitude monophasic pulses (of, for example, a pulse duration of 50 µS). In another embodiment, a continuous series of, for example, 60 biphasic high-amplitude, (of, for example, greater than 1000 V), short-pulse-duration pulses may be delivered, followed by a series of low-amplitude (of, for example, less than 200 V) monophasic pulses. The lower amplitude pulses may be monophasic or biphasic with a direct current (DC) offset, or unbalanced biphasic pulses, such that they impart charge to the target tissue. Within the affected target tissue, this DC current may produce changes in the tissue's acid-base and may produce certain active oxygen species that may enhance and increase targeted cell death. That is, the delivery of DC current through the tissue may result in electrolysis, which produces chemically active molecules that enhance tissue necrosis in the region of tissue in which they are generated. Different chemical reactions take place at the anode and at the cathode, which may result in different chemical species produced at each. The anode produces oxidative reactions and a low pH, while the cathode produces a high-pH, reducing chemical environment. Additionally, in alternating or subsequent pulse combinations, the charge balance may be reversed and/or reduced with subsequent deliveries in the procedure creating competing cytotoxic species that act differently for effecting cell death or competing with remaining species at the effective positive or negative electrode locations (subsequently reversed) to cease or minimize further damage from the initial electrolyzing. A complex variety of peroxides, hydroperoxides, nitrogen oxides, chlorates, as well as reactive compounds containing phosphorous, sulfur nitrogen, and/or various metal ion components may be formed, which are highly cytotoxic. The effects of these chemical species may be enhanced by inserting the treatment electrode(s) 40 into the tissue, such as by those configurations shown in FIGS. 1, 2, 4, and 6. This method of energy delivery may enhance lesion formation without arrhythmogenic consequences within relatively thick target tissues, such as in the ventricles. The generator 20 may also be configured to deliver similar, but of lower-amplitude, charge-neutral pulses to stun and/or ablate the target tissue. Extremely short (for example, between approximately 0.5 to 1 µS in duration), lower-amplitude pulses delivered at a rate of between approximately 0.1 and 1.0 MHz may have the potential to impart charge to a target tissue site, thereby ablating at least a portion of the target tissue site and stunning at least a portion of the target tissue site beyond the ablated portion. This cellular inactivation may help prevent the inducement of arrhythmia in the tissue as a result of subsequent ablation energy delivery.

The generator 20 may also be configured to deliver energy for heating tissue, such as relatively low voltage AC pulsed currents (of, for example, less than 400 V), continuous RF, and/or pulsed RF or microwave energy, before, during, or after delivering monophasic and/or biphasic electroporation energy.

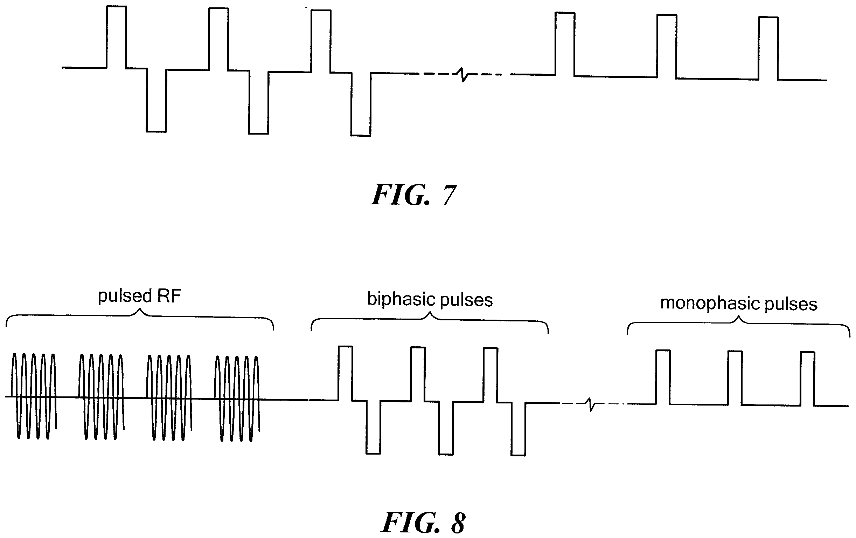

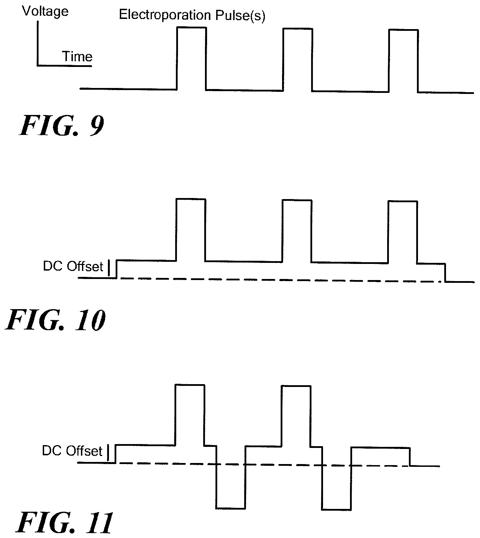

FIGS. 7-9 show waveforms of energy that may be delivered to induce electroporation of myocardial cells. The waveforms represent voltage delivered (y-axis) over time (x-axis). FIG. 7 shows a configuration that imparts charge to the tissue by omission of the reversed biphasic components later in a pulse train or in subsequently applied trains, as long as the net charge, relative positive charge versus negative charge, is sufficiently different between the phases to result in aggregated charge in the target tissue. FIG. 8 shows exemplary pulsed RF energy delivery to warm tissue, then biphasic energy pulses for stunning and/or ablating the tissue, and monophasic pulses delivered thereafter for inducing electrolysis (to further ablate the tissue). FIG. 9 shows a waveform of a typical monophasic energy delivery.

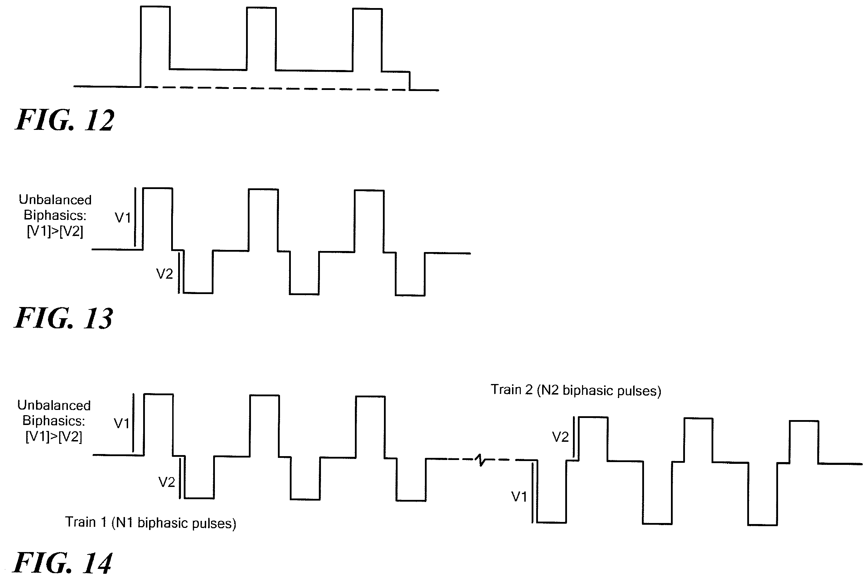

FIGS. 10-14 show exemplary waveforms of biphasic and/or monophasic energy pulses used as discussed herein to induce or enhance electrolysis without inducing further or additional cardiac arrhythmia. The waveforms shown in FIGS. 10-14 include a DC offset as discussed herein and/or different ways of unbalancing the delivered charge, whether in the same train or sequentially. For example, FIG. 10 shows a monophasic waveform that includes a DC offset, FIG. 11 shows a biphasic waveform that includes a DC offset, FIG. 12 shows a monophasic waveform with concurrent delivery of DC energy, FIG. 13 shows an asymmetric biphasic waveform, and FIG. 14 shows an asymmetric biphasic waveform in which the relative anode versus cathode is reversed.

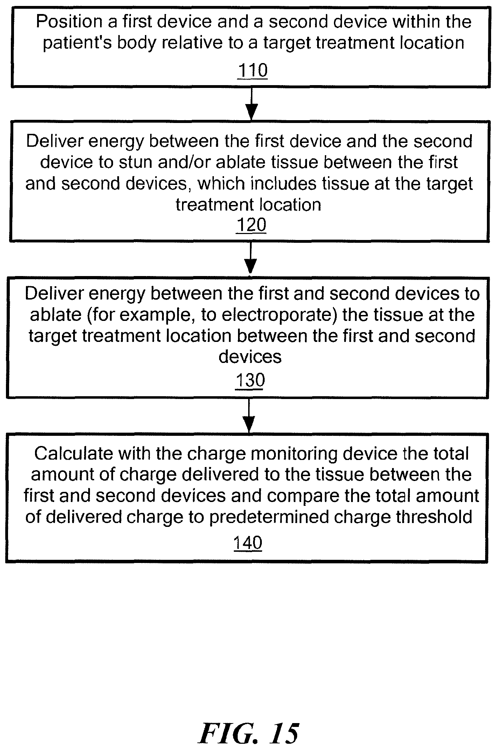

Turning to FIGS. 15-18, various methods of destroying target tissue through electroporation are shown generally and described. In general, these methods involve one or more steps to "prime" or optimize the target tissue for electroporation, thereby increasing the depth and volume of tissue that is ablated by delivery of electroporation energy. For example, this tissue optimization may involve the delivery of biphasic, non-charge-neutral energy to create toxic chemical byproducts and/or may involve the application of energy, such as pulsed RF energy, to heat the tissue. Both or either of which may lower the threshold electric field strength at which cells will incur irreversible membrane damage and, therefore, cell death. This, in turn, may reduce the amount of voltage that is required to induce irreversible electroporation and reduce the likelihood of device and/or generator faults or failure. Further, one or both of these methods of tissue optimization may be used in a given procedure, and they may be used before, during, and/or after the delivery of electroporation energy. For example, cells may experience a period of increased permeability even after the delivery of electroporation energy has ended. Therefore, the creation of toxic chemical byproducts and/or application of heat to electroporated tissue may still enhance overall treatment results.

Referring to FIG. 15, a general method of treating tissue with one or two devices is shown. In the first step 110, a first device 12 and a treatment 14 device may be positioned within the patient's body at one or more locations that will result in the delivery of treatment energy to tissue at the target treatment location. For example, the devices 12, 14 may be treatment devices. In a second step 120, energy may be delivered between the first 12 and second 14 devices to optimize tissue at the target treatment location for ablation. In one embodiment, energy may be delivered between the first 12 and second 14 devices to stun and/or ablate the tissue between the first 12 and second 12 devices, which includes tissue at the target treatment location. For example, extremely short, biphasic, high-amplitude, high-voltage, charge-neutral pulses may be delivered to tissue at the target treatment site. In addition to reducing the risk of generating an arrhythmia with the delivery of charge-neutral ablation energy, this energy delivery may be used to determine optimal target ablation sites before commencement of the delivery of additional ablation energy. The energy delivered in the second step 120 does not impart charge to the tissue. Additionally or alternatively, in another embodiment, pulsed RF energy may be delivered between the first 12 and second 14 devices to heat the tissue to a temperature that is generally lower than a temperature at which hypothermal tissue ablation occurs, but to a temperature that is high enough to enhance tissue ablation by electroporation.

In the third step 130, energy may be delivered between the first 12 and second 14 devices to ablate or further ablate, such as electroporate, the tissue at the target treatment location. The second step 120 is shown in FIG. 15 as occurring before the third step 130 for simplicity; however, the second step 120 may occur before, during, and/or after the third step 130. In some embodiments, the second 120 and third 130 steps may occur as a single treatment step. For example, the single treatment step combining the second 120 and third 130 steps may include using the first 12 and second 14 devices to deliver high-amplitude, short-pulse-duration, biphasic, charge-neutral pulses combined with a series of lower-amplitude, non-charge-neutral pulses delivered between the high-amplitude pulses, in order to electroporate the target tissue and to induce formation of cytotoxic chemical species to be taken up by the permeabilized cells.

In an optional fourth step 140, the charge monitoring device 46 may calculate the total amount of energy delivered to the target tissue throughout the procedure. Thus, the delivered energy may be recorded at all stages of the procedure in which energy is delivered to the tissue. The processing circuitry 44 may be configured to establish or determine a total amount of delivered energy at which the processing circuitry 44 may automatically cease the delivery of ablation energy or at which the system 10 will alert the user to manually end the delivery of ablation energy (which may be referred to herein as a predetermined charge threshold). If the total amount of delivered energy is equal to or greater than the predetermined charge threshold, the processing circuitry 44 may automatically cease the delivery of ablation energy or will alert the user to manually end the delivery of ablation energy. Further, the processing circuitry 44 may be configured to use data about the total amount of delivered energy to confirm that the required DC offset is being delivered, to confirm that the delivered charge is not excessive enough to obscure EGM recordings and/or to provide feedback to the user that the user may expect a transient impact on the EGM recordings of an EP device, and/or to confirm that the total amount of delivered charge is not excessive (for example, that an amount capable of causing arrhythmia or death is not being delivered to the patient).

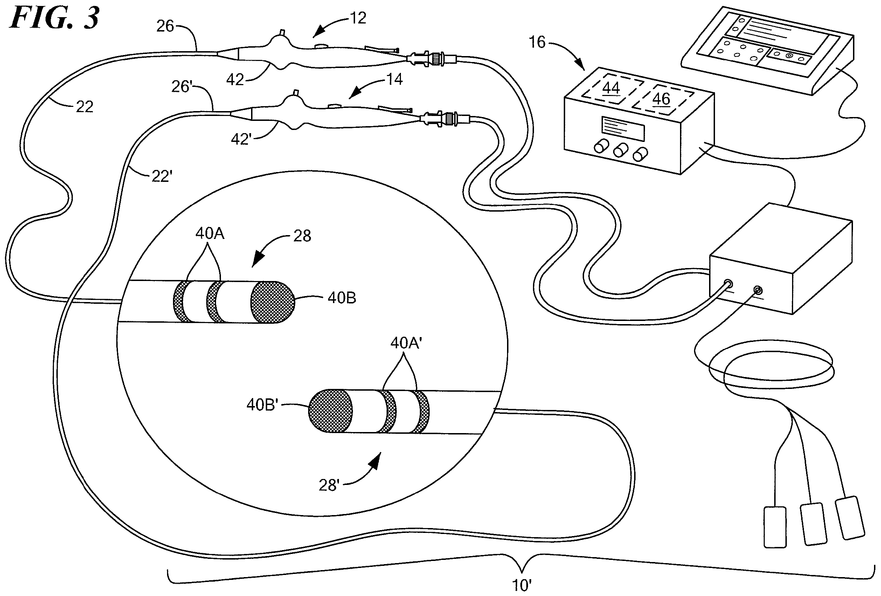

Although FIG. 15 shows a method using two devices 12, 14, the method may be performed using only one device 12. In that case, the monophasic deliveries may be delivered in a bipolar manner. To accomplish this, the device may include more than one electrode 40 (for example, the flat electrodes as shown in FIG. 3), and adjacent electrodes 40 may have opposite polarities. However, when the intent is to create electrochemical products of the direct electric current, some physical separation may be maintained between the electrode(s) serving as the anode(s) and the electrode(s) serving as the cathode(s), such that the chemical moieties created at the anode(s) remain relatively stagnant in the tissue and unable to readily mix with the chemical milieu created at the cathodic electrode(s). This may allow for the greatest tissue cytotoxic effect from the locally created chemicals. Additionally, one or both anode and cathode electrodes may be embedded in some way within the tissue. For example, if the electrode 40 is in the form of a needle (such as that shown in FIGS. 1 and 2), the electrode 40 may be able to penetrate into the tissue, or an electrode of the device 12 may be delivered into the lumen of a distal artery where the blood perfusion flow targets the desired area to be ablated.

As a first non-limiting example of a method of treatment according to FIG. 15, the first treatment device 12 may be positioned within the heart on an endocardial surface. If the first treatment device 12 includes an electrode 40 that is configured to be inserted into the tissue, the distal portion of the device and the electrode 40 may be manipulated to insert or screw the electrode into the target tissue. Alternatively, if the device 12 includes one or more flat electrodes 40, the distal portion of the device may be positioned such that the flat electrodes 40 are in contact with the target tissue. The second treatment device 14 may be positioned on the opposing wall of the heart from the location of the first treatment device 12, in a different heart chamber. The electrode(s) 40 of the second treatment device 14 may be positioned similar to those of the first treatment device 12. One of the two treatment devices may serve as an anode and the other of the two treatment devices may serve as a cathode. However, when a biphasic pulse is delivered, each may serve as the anode and cathode during some phase of the energy delivery (that is, the roles of anode and cathode may alternate during biphasic pulse delivery). Pulsed DC energy may be delivered between the two devices 12, 14, thus producing electrolysis within the intervening tissues.

In a second non-limiting example of a method of treatment according to FIG. 15, the first treatment device 12 may be positioned within the patient's body at one or more locations that will result in the delivery of treatment energy in the target treatment site(s). As a non-limiting example, the first treatment device 12 may be positioned within the heart on an endocardial surface. If the first treatment device 12 includes an electrode 40 that is configured to be inserted into the tissue, the distal portion of the device and the electrode 40 may be manipulated to insert or screw the electrode into the target tissue. Alternatively, if the device 12 includes one or more flat electrodes 40, the distal portion of the device may be positioned such that the flat electrodes 40 are in contact with the target tissue. The second treatment device 14 may be positioned within a coronary arterial or venous blood vessel. Pulsed DC energy may be delivered between the two devices 12, 14, thus producing electrolysis within the intervening tissues. The current flow could be preferentially directed such that the device functioning as the anode is located closest to the tissue targeted for ablation.

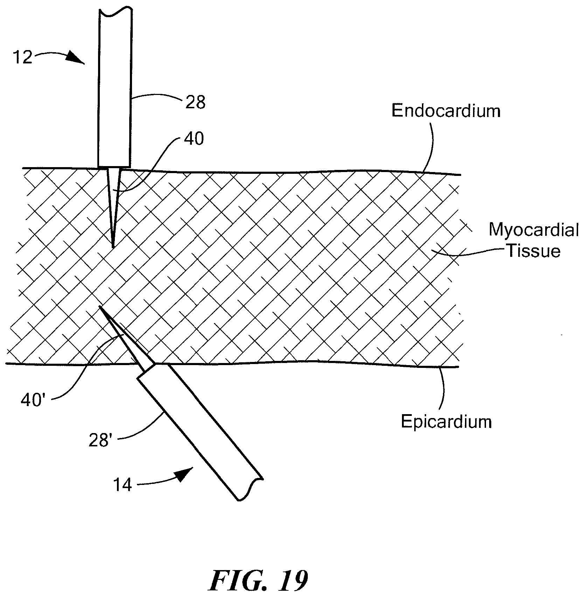

In a third example of a method of treatment according to FIG. 15, the first treatment device 12 may be positioned within the patient's body at one or more locations that will result in the delivery of treatment energy in the target treatment site(s). As a non-limiting example, the first treatment device 12 may be positioned within the heart on an endocardial surface. If the first treatment device 12 includes an electrode 40 that is configured to be inserted into the tissue, the distal portion of the device and the electrode 40 may be manipulated to insert or screw the electrode into the target tissue. Alternatively, if the device 12 includes one or more flat electrodes 40, the distal portion of the device may be positioned such that the flat electrodes 40 are in contact with the target tissue. The second treatment device 14 may be positioned within the pericardial space, in contact with the epicardium. Likewise, if the first treatment device 12 includes an electrode 40 that is configured to be inserted into the tissue, the distal portion of the device and the electrode 40 may be manipulated to insert or screw the electrode into the target tissue. A non-limiting example of device 12, 14 positioning is shown in FIG. 19, in which a first device 12 is located proximate the endocardial surface and includes an invasive electrode 40 that is inserted into the myocardial tissue, and the second device 14 is located proximate the epicardial surface or within the pericardial space and also includes an invasive electrode 40' that is inserted into the myocardial tissue. Pulsed DC energy may be delivered between the two devices 12, 14, thus producing electrolysis within the intervening tissues. The current flow could be preferentially directed such that the device functioning as the anode is located closest to the tissue targeted for ablation.

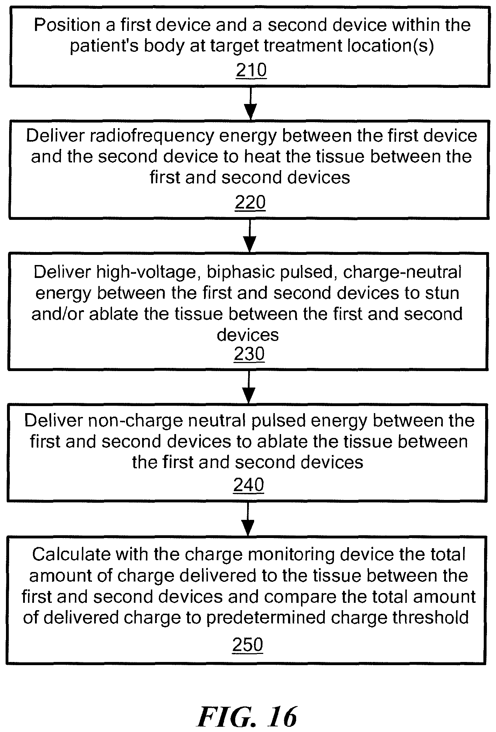

Referring now to FIGS. 16-18, exemplary methods are shown and described in more detail. A method of treating tissue using two devices and optimizing the tissue using heat is shown in FIG. 16. In addition to or instead of optimization method discussed above in the third step 130 of FIG. 15, one or both devices 12, 14 may be used to deliver pulsed RF energy for a predetermined time period, the predetermined time being sufficient to heat the cells to a target temperature. Heating the tissue may reduce the threshold electric field strength of the tissue that is required to cause irreversible cell membrane damage. The temperature increase required, and thus the target temperature, to achieve increased electroporation effectiveness may be less than the minimum temperature that would be required to achieve cell death by thermal means alone (approximately 50 °C). Further, in circulating blood, an electrode-tissue interface temperature of approximately 60 °C is accepted as not producing blood protein denaturation or other injurious effects. In one embodiment, the tissue may be optimized by heating to a temperature of at least approximately 45° C and as high as approximately 60 °C. That is, the tissue may be heated to a temperature of between approximately 45 °C and approximately 60°C. At these temperatures, the sub-lethal heat may be driven as deep as possible into the target tissue, which may thereby increase the depth of electroporation ablation. To heat the tissue, the device(s) may deliver relatively low voltage AC pulsed currents, continuous RF or pulsed RF or microwave energy. After the first and second devices 12, 14 may be positioned at one or more target treatment locations in the first step 210, one or both device(s) 12, 14 may be used to deliver (for example, to deliver simultaneously) pulsed RF energy to heat the cells at the target treatment location in the second step 220 prior to ablation. For example, the energy delivered in the second step 220 may have the waveform shown in the first portion of FIG. 8. In one embodiment, the pulsed RF energy is delivered for a predetermined time before the delivery of the non-charge-neutral pulses and the charge-neutral pulses, the predetermined period of time being sufficient to heat the tissue to a temperature of at least approximately 45° C and up to approximately 60 °C. In another embodiment, the generator includes a feedback system that monitors a temperature recorded by one or both device(s) 12, 14 to ensure the tissue is not overheated above a temperature of at least approximately 45 °C and up to approximately 60 °C.

In the third step 230, high-voltage, short-pulse-duration, biphasic, charge-neutral pulses may be delivered between the devices 12, 14 to incapacitate the tissue between the devices 12, 14 at the target treatment location. In the fourth step 240, non-charge-neutral pulsed energy may then be delivered between the devices 12, 14 to ablate the tissue located between the devices 12, 14 at the target treatment location. Additional pulsed RF energy may optionally be delivered after all delivery of electroporation energy has ended. The third step 230 is shown in FIG. 16 as occurring before the fourth step 240 for simplicity; however, the third step 130 may occur before, during, and/or after the fourth step 240. In some embodiments, the third 230 and fourth 240 steps may occur as a single treatment step. For example, the high-voltage, short-pulse-duration, biphasic, charge-neutral pulses of the third step 230 may be delivered sequentially with, simultaneously with, or alternating with the series of lower-amplitude, non-charge-neutral pulses of the fourth step 240.

In an optional fifth step 250, the charge monitoring device 46 may calculate the total amount of energy delivered to the tissue at the target treatment location throughout the procedure and compare the total amount of delivered energy to the predetermined charge threshold. Thus, the delivered energy may be recorded at all stages of the procedure in which energy is delivered to the tissue. The processing circuitry 44 may be configured to establish or determine a total amount of delivered energy at which the processing circuitry 44 may automatically cease the delivery of ablation energy or at which the system 10 will alert the user to manually end the delivery of ablation energy (which may be referred to herein as a predetermined charge threshold). If the total amount of delivered energy is equal to or greater than the predetermined charge threshold, the processing circuitry 44 may automatically cease the delivery of ablation energy or will alert the user to manually end the delivery of ablation energy. Further, the processing circuitry 44 may be configured to use data about the total amount of delivered energy to confirm that the required DC offset is being delivered, to confirm that the delivered charge is not excessive enough to obscure EGM (intracardiac electrogram) recordings and/or to provide feedback to the user that the user may expect a transient impact on the EGM recordings of an EP device, and/or to confirm that the total amount of delivered charge is not excessive (for example, that an amount capable of causing arrhythmia or death is not being delivered to the patient).

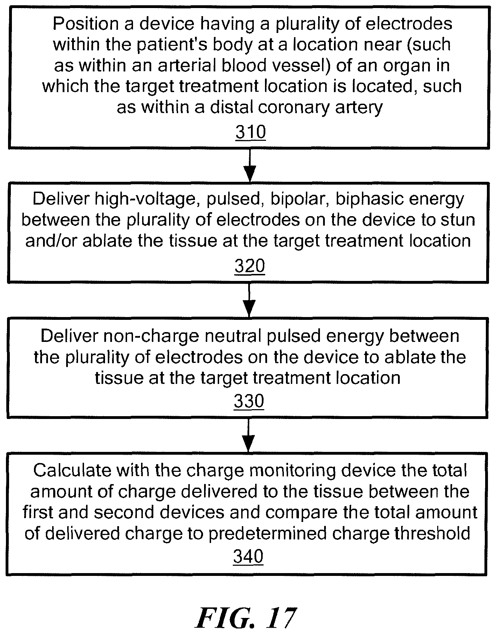

Referring now to FIG. 17, a flow chart for a further exemplary method of delivery energy to tissue is shown. In the method of FIG. 17, a single device 12 having a plurality of electrodes 40 is used at a location remote from the target treatment location. Adjacent electrodes 40 of the plurality of electrodes 40 may have opposite polarities. In the first step 310, the device 12 may be positioned within the patient's body at one or more locations that will result in the delivery of treatment energy in the target treatment site(s). In one embodiment, the device 12 is positioned within an arterial blood vessel of the heart, such as within a distal coronary artery, proximate a target treatment location within the heart. In the second step 320, high-voltage, short-pulse-duration, biphasic, charge-neutral pulses may be delivered between the plurality of electrodes 40 12 to stun and/or ablate the tissue. The coronary arteries extend from the aorta to the outside of the heart, thereby supplying blood to the heart. The device 12 may be positioned within a coronary artery, and any toxins produced by the delivery of energy by the device 12 at this location may have a desired effect on the tissue at the target treatment location.

In the third step 330, the device 12 may deliver lower-amplitude, non-charge-neutral, pulsed energy between the plurality of electrodes 40 while in place within the coronary artery to ablate the tissue at the target treatment location. In an optional fourth step 340, the charge monitoring device 46 may calculate the total amount of energy delivered to the tissue at the target treatment location throughout the procedure and compare the total amount of delivered energy to the predetermined charge threshold. If the total amount of delivered energy is equal to or greater than the predetermined charge threshold, the processing circuitry 44 may automatically cease the delivery of ablation energy or will alert the user to manually end the delivery of ablation energy.

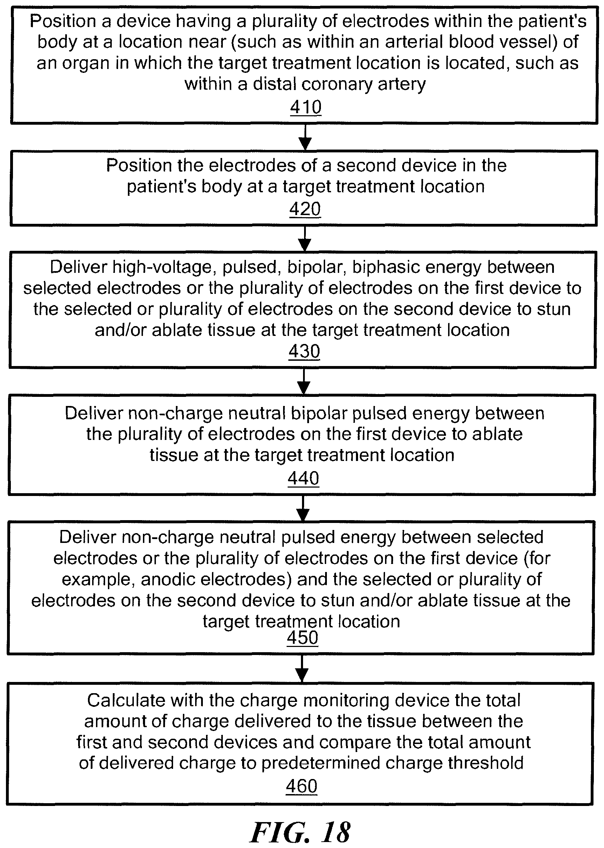

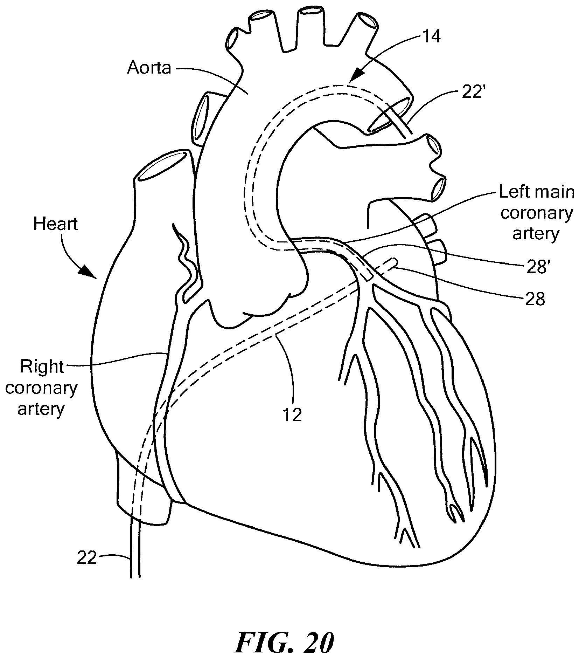

Referring now to FIG. 18, a flow chart for a further exemplary method of delivery energy to tissue is shown. In the method of FIG. 17, a first device 12 is used at a location remote from the target treatment location and a second device 14 is used at the target treatment location. In the first step 410, the device 12 may be positioned within the patient's body at one or more locations that will result in the delivery of treatment energy in the target treatment site(s). In one embodiment, the device 12 is positioned within an arterial blood vessel of the heart, such as within a distal coronary artery, proximate a target treatment location within the heart. In the second step 420, the second device 14 is positioned at or proximate the target treatment location. Each of the first 12 and second 14 devices may include one or a plurality of electrodes 40. In one embodiment, the target treatment location may be at a location within the left ventricle of the heart. A non-limiting example of device 12, 14 placement is shown in FIG. 20, in which the first device 12 is within the left ventricle and the second device 14 is within the coronary artery.

In a third step 430, high-voltage, short-pulse-duration, biphasic, charge-neutral pulses may be delivered between first 12 and second 14 devices to stun and/or ablate tissue between the first 12 and second 14 devices, which may include tissue at the target treatment location. For example, energy may be delivered between selected one or more of the plurality of electrodes 40 on the first device and one or more electrodes of the plurality of electrodes 40 on the second device 14 to stun and/or ablate tissue at the target treatment location. In the fourth step 440, lower-amplitude, non-charge-neutral, pulsed energy may be delivered from or between one or more of the plurality of electrodes 40 of the second device 14 to ablate the tissue at the target treatment location. In the fifth step 450, high-voltage, short-pulse-duration, non-charge-neutral energy may be delivered between one or more of the plurality of electrodes 40 of the first device 12 (for example, anodic electrodes) and one or more of the plurality of electrodes 40 of the second device 14 to further stun and/or ablate tissue at the target treatment location. In an optional sixth step 460, the charge monitoring device 46 may calculate the total amount of energy delivered to the tissue at the target treatment location throughout the procedure and compare the total amount of delivered energy to the predetermined charge threshold. If the total amount of delivered energy is equal to or greater than the predetermined charge threshold, the processing circuitry 44 may automatically cease the delivery of ablation energy or will alert the user to manually end the delivery of ablation energy.

As a non-limiting example, the first treatment device 12 may be positioned within the heart on an endocardial surface. If the first treatment device 12 includes an electrode 40 that is configured to be inserted into the tissue, the distal portion of the device and the electrode 40 may be manipulated to insert or screw the electrode into the target tissue. Alternatively, if the device 12 includes one or more flat electrodes 40, the distal portion of the device may be positioned such that the flat electrodes 40 are in contact with the target tissue. The second treatment device 14 may be positioned on the opposing wall of the heart from the location of the first treatment device 12, in a different heart chamber. The electrode(s) 40 of the second treatment device 14 may be positioned similar to those of the first treatment device 12. One of the two treatment devices may serve as an anode and the other of the two treatment devices may serve as a cathode. However, when a biphasic pulse is delivered, each may serve as the anode and cathode during some phase of the energy delivery (that is, the roles of anode and cathode may alternate during biphasic pulse delivery). Pulsed DC energy may be delivered between the two devices 12, 14, thus producing electrolysis within the intervening tissues.

The devices, systems, and methods disclosed herein may be used to treat existing cardiac arrhythmias without inducing further or additional arrhythmias. To accomplish this, short pulses of energy may be delivered to the target tissue, and biphasic pulses may be delivered initially to immobilize the underlying myocytes. A localized charge imbalance may be induced to create a local toxic chemical environment, but that creates an overall charge balance within the heart. Further, alternating anode/cathode energy delivery configurations may be used to enhance cell death using one, and then the other electrode polarity to produce both chemical species at each electrode site. The system may also be used to monitor and control the amount of charge delivered to the tissue. As a non-limiting example, the processing circuitry 44 may configured to calculate a total amount of charge delivered to the target tissue during the entire treatment procedure, or at least a portion of the treatment procedure, and to automatically adjust the level of pulse imbalance to maintain a desired or predetermined charge level. The total amount of charge may be based on, for example, the number and duration of non-charge-neutral pulses delivered to the target tissue.

The devices, systems, and methods disclosed herein may also be used to enhance the effects of ablation. For example, a needle-shaped or helical-shaped energy delivery electrode may be used to deliver hypertonic and ionic solution(s) to the target tissue site. Further, one or more invasive electrodes may be inserted into the myocardial tissue, thereby producing localized lethal chemical products deeper within the myocardium. Still further, the system and device(s) may be used to produce electrolysis products within a distal arterial supply, which products may then travel to the target myocardial tissue to enhance the effects of the cardiac ablation. Combinations of any or all of these techniques may be used to, for example, deliver energy between electrodes in arteries, between and/or from invasive electrodes, and/or between and/or from endocardial electrodes and/or epicardial electrodes.

It will be appreciated by persons skilled in the art that the present invention is not limited to what has been particularly shown and described herein above. In addition, unless mention was made above to the contrary, it should be noted that all of the accompanying drawings are not to scale. A variety of modifications and variations are possible in light of the above teachings without departing from the scope of the invention, which is limited only by the following claims.

## Claims

1. A medical system (10), the system comprising:
a first treatment device (12) including at least one treatment electrode (40) selected from the group consisting of a needle-shaped electrode and a helical-shaped electrode;
a second treatment device (14) including at least one treatment electrode (40') selected from the group consisting of a needle-shaped electrode and a helical-shaped electrode; and
an energy generator (16) in communication with the first and second treatment devices, the energy generator being programmed to:
deliver charge-neutral pulses; **characterized in that** the energy generator is further programmed to
deliver non-charge-neutral pulses between the charge-neutral pulses, wherein
the charge-neutral pulses are delivered at a first amplitude and the non-charge-neutral pulses being delivered at a second amplitude, the first amplitude being greater than the second amplitude.

2. The system of Claim 1, wherein the non-charge-neutral pulses are one of monophasic and biphasic.

3. The system of Claim 2, wherein the non-charge-neutral pulses have a direct current offset.

4. The system of Claim 3, wherein the system is configured to deliver charge-neutral and non-charge neutral pulses to an area of target tissue, delivery of the non-charge-neutral pulses imparting a charge to the target tissue.

5. The system of any of Claims 1 3 or 4, wherein the energy generator (16) is further programmed to deliver non-charge-neutral pulses at a third amplitude, the third amplitude being less than each of the first and the second amplitudes.

6. The system of any of Claims 1, 3 or 4, wherein the at least one treatment electrode (40,40') is configured to be inserted into an area of target tissue.

7. The system of Claim 6, wherein the at least one treatment electrode (40) on the first treatment device (12) is a needle-shaped electrode.

8. The system of Claim 6, wherein the at least one treatment electrode (40') on the second treatment device is a helical-shaped electrode.

9. The system of Claim 6, wherein the at least one treatment electrode (40,40') is in fluid communication with a fluid source, the at least one treatment electrode including a plurality of apertures configured to deliver fluid from the fluid source to the area of target tissue.

10. The system of any of Claims 1, 3, 4 or 7 to 9, wherein the energy generator (16) is further programmed to deliver pulsed radiofrequency energy one of concurrently with or independently from the delivery of the non-charge-neutral pulses and the charge-neutral pulses.

11. The system of Claim 10, wherein the pulsed radiofrequency energy is one of unipolar and bipolar, the pulsed radiofrequency energy being delivered for a predetermined period of time before the delivery of the non-charge-neutral pulses and the charge-neutral pulses, the predetermined period of time being sufficient to heat the tissue to a temperature that is lower than a temperature at which tissue ablation occurs.

## Patentansprüche

1. Medizinisches System (10), wobei System Folgendes umfasst:
eine erste Behandlungsvorrichtung (12), die mindestens eine Behandlungselektrode (40) aufweist, die aus der Gruppe bestehend aus einer nadelförmigen Elektrode und einer spiralförmigen Elektrode ausgewählt ist;
eine zweite Behandlungsvorrichtung (14), die mindestens eine Behandlungselektrode (40') aufweist, die aus der Gruppe bestehend aus einer nadelförmigen Elektrode und einer spiralförmigen Elektrode ausgewählt ist; und
einen Energiegenerator (16) in Verbindung mit der ersten und der zweiten Behandlungsvorrichtung, wobei der Energiegenerator programmiert ist zum:
Abgeben ladungsneutraler Impulse; **dadurch gekennzeichnet, dass** der Energiegenerator ferner programmiert ist zum:
Abgeben nicht ladungsneutraler Impulse zwischen den ladungsneutralen Impulsen, wobei die ladungsneutralen Impulse mit einer ersten Amplitude abgegeben werden und die nicht ladungsneutralen Impulse mit einer zweiten Amplitude abgegeben werden, wobei die erste Amplitude größer als die zweite Amplitude ist.

2. System nach Anspruch 1, wobei die nicht ladungsneutralen Impulse monophasig oder zweiphasig sind.

3. System nach Anspruch 2, wobei die nicht ladungsneutralen Impulse einen Gleichstrom-Offset aufweisen.

4. System nach Anspruch 3, wobei das System dazu ausgelegt ist, ladungsneutrale und nicht ladungsneutrale Impulse an einen Bereich eines Zielgewebes abzugeben, wobei die Abgabe der nicht ladungsneutralen Impulse dem Zielgewebe eine Ladung verleiht.

5. System nach einem der Ansprüche 1, 3 oder 4, wobei der Energiegenerator (16) ferner dazu programmiert ist, nicht ladungsneutrale Impulse mit einer dritten Amplitude abzugeben, wobei die dritte Amplitude kleiner als jede von der ersten und der zweiten Amplitude ist.

6. System nach einem der Ansprüche 1, 3 oder 4, wobei die mindestens eine Behandlungselektrode (40, 40') dazu ausgelegt ist, in einen Bereich eines Zielgewebes eingesetzt zu werden.

7. System nach Anspruch 6, wobei die mindestens eine Behandlungselektrode (40) auf der ersten Behandlungsvorrichtung (12) eine nadelförmige Elektrode ist.

8. System nach Anspruch 6, wobei die mindestens eine Behandlungselektrode (40') auf der zweiten Behandlungsvorrichtung (12) eine spiralförmige Elektrode ist.

9. System nach Anspruch 6, wobei die mindestens eine Behandlungselektrode (40,40') mit einer Fluidquelle in Fluidverbindung steht, wobei die mindestens eine Behandlungselektrode eine Mehrzahl von Öffnungen aufweist, die dazu ausgelegt ist, Fluid aus der Fluidquelle an den Bereich des Zielgewebes abzugeben.

10. System nach einem der Ansprüche 1, 3, 4 oder 7 bis 9, wobei der Energiegenerator (16) ferner dazu programmiert ist, gepulste Hochfrequenzenergie gleichzeitig mit oder unabhängig von der Abgabe der nicht ladungsneutralen Impulse und der ladungsneutralen Impulse abzugeben.

11. System nach Anspruch 10, wobei die gepulste Hochfrequenzenergie unipolar oder bipolar ist, die gepulste Hochfrequenzenergie für einen vorgegebenen Zeitraum vor der Abgabe der nicht ladungsneutralen Impulse und der ladungsneutralen Impulse abgegeben wird und der vorgegebene Zeitraum ausreicht, um das Gewebe auf eine Temperatur zu erwärmen, die niedriger als eine Temperatur ist, bei der Gewebeablation stattfindet.

## Revendications

1. Système médical (10), le système comprenant :
un premier dispositif de traitement (12) comportant au moins une électrode de traitement (40) choisie dans le groupe constitué par une électrode en forme d'aiguille et une électrode de forme hélicoïdale ;
un deuxième dispositif de traitement (14) comportant au moins une électrode de traitement (40') choisie dans le groupe constitué par une électrode en forme d'aiguille et une électrode de forme hélicoïdale ; et
un générateur d'énergie (16) en communication avec les premier et deuxième dispositifs de traitement, le générateur d'énergie étant programmé pour :
délivrer des impulsions de charge neutre ; **caractérisé en ce que** le générateur d'énergie est également programmé pour
délivrer des impulsions de charge non neutre entre les impulsions de charge neutre, dans lequel les impulsions de charge neutre sont délivrées à une première amplitude et les impulsions de charge non neutre sont délivrées à une deuxième amplitude, la première amplitude étant supérieure à la deuxième amplitude.

2. Système selon la revendication 1, dans lequel les impulsions de charge non neutre sont soit monophasiques, soit biphasiques.

3. Système selon la revendication 2, dans lequel les impulsions de charge non neutre ont un décalage de courant continu.

4. Système selon la revendication 3, le système étant conçu pour délivrer des impulsions de charge neutre et de charge non neutre à une zone de tissu cible, la délivrance des impulsions de charge non neutre conférant une charge au tissu cible.

5. Système selon l'une quelconque des revendications 1, 3 et 4, dans lequel le générateur d'énergie (16) est également programmé pour délivrer des impulsions de charge non neutre à une troisième amplitude, la troisième amplitude étant inférieure à chacune des première et deuxième amplitudes.

6. Système selon l'une quelconque des revendications 1, 3 et 4, dans lequel l'au moins une électrode de traitement (40, 40') est conçue pour être insérée dans une zone de tissu cible.

7. Système selon la revendication 6, dans lequel l'au moins une électrode de traitement (40) sur le premier dispositif de traitement (12) est une électrode en forme d'aiguille.

8. Système selon la revendication 6, dans lequel l'au moins une électrode de traitement (40') sur le deuxième dispositif de traitement est une électrode de forme hélicoïdale.

9. Système selon la revendication 6, dans lequel l'au moins une électrode de traitement (40, 40') est en communication fluidique avec une source de fluide, l'au moins une électrode de traitement comportant une pluralité d'ouvertures conçues pour délivrer un fluide provenant de la source de fluide à la zone de tissu cible.

10. Système selon l'une quelconque des revendications 1, 3, 4 et 7 à 9, dans lequel le générateur d'énergie (16) est également programmé pour délivrer de l'énergie radiofréquence pulsée soit simultanément à, soit indépendamment de la délivrance des impulsions de charge non neutre et des impulsions de charge neutre.

11. Système selon la revendication 10, dans lequel l'énergie radiofréquence pulsée est soit unipolaire, soit bipolaire, l'énergie radiofréquence pulsée étant délivrée pendant un laps de temps prédéterminé avant la délivrance des impulsions de charge non neutre et des impulsions de charge neutre, le laps de temps prédéterminé étant suffisant pour chauffer le tissu jusqu'à une température qui est inférieure à une température à laquelle une ablation de tissu se produit.
